# EUROPEAN PATENT APPLICATION

(11) **EP 1 300 166 A1**
(43) Date of publication of application: **09.04.2003**
(21) Application number: 02256688.9
(22) Date of filing: 25.09.2002
(51) Int. Cl.: A61L 31/16, A61L 31/10

(54) **Means for treatment of coronary artery obstructions**

(30) Priority: 02.10.2001 US 969165
(71) Applicant: Cordis Corporation, Miami Lakes Florida 33014 (US)
(72) Inventor: Fischell, Robert E., Dayton, MD 21036 (US); Fischell, David R., Fair Haven, NJ 07704 (US); Fischell, Tim A., Richland, MI 49083 (US)
(74) Representative: Belcher, Simon James

(57) **Abstract**

A stent for implantation into an artery of a human subject, the stent comprises a thin-walled, lace-like, metal structure formed into the general shape of a cylindrical tube. The stent has a drug coating on its surface, the drug being an anti-restenosis drug selected from the group that includes, Alkeran, Cytoxan, Leukeran, BiCNU, Cerubidine, Fluorouracil, Methotrexate, Toxotere, Irinotecan, Hycamptin, Matulane, Vumon, Hexalin, Gemzar, Oncovin, Etophophos.

## Description

This invention is in the field of stents to create and maintain patency of vessels of a human body.

Stents have been placed in the arteries of human subjects for more than ten years. A continuing problem for these devices is that restenosis occurs in many patients, particularly when the stent is implanted in a peripheral or coronary artery. One solution to this problem has been to coat stents with a drug that oozes out from the stent after it has been placed in an artery. As of the year 2001, stents coated with Taxol or Rapamycin have been used to decrease the rate of restenosis after stent implantation. However, there are many other medications that can act with the same or increased efficacy as compared to Taxol or Rapamycin.

A significant need is for stents to have a coating that prevents subacute thrombosis as well as restenosis. Although some stents with heparin type coatings have been used in human subjects, no stent has combined a drug coating such as Taxol or Rapamycin with a heparin type coating to both decrease the rate of restenosis and decrease the rate of subacute thrombosis.

A preferred embodiment of this invention is a stent that is coated with an anti-restenosis drug that is selected from the group that includes, Alkeran, Cytoxan, Leukeran, Cis-platinum, BiCNU, Adriamycin, Doxorubicin, Cerubidine, Idamycin, Mithracin, Mutamycin, Fluorouracil, Methotrexate, Thoguanine, Toxotere, Etoposide, Vincristine, Irinotecan, Hycamptin, Matulane, Vumon, Hexalin, Hydroxyurea, Gemzar, Oncovin, Etophophos, tacrolimus (FK506), and the following analogs of sirolimus: SDZ-RAD, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy, 2-desmethyl and proline.

Typically, the drug would be placed onto or into a plastic coating such as paralene that is placed onto a metal stent. Drug coated stents could be used for a multiplicity of coronary arteries that would otherwise require the patient to have coronary artery bypass surgery. Explicitly, it is conceived that drug coated stents could be placed in at least three and as many as five coronary arteries that have diffuse and/or severe restenosis. As many as two stents per artery could be used for this purpose.

Another embodiment of this invention is to have a stent that has an anti-restenosis drug coated on its outer surface that is placed in contact with the arterial wall and the inner surface through which the blood flows is coated with an anti-thrombogenic coating such as heparin or phosphorocolene. Still further it is conceived that a stent could combine both an anti-restenosis drug and an anti-thrombogenic drug in a single coating that coats the entire surface of a stent.

Embodiments of the invention will now be described by way of example with reference to the accompanying drawings, in which:
FIG. 1 is a cross section of a strut of a conventional, prior art, metal stent as is well known in the art of interventional cardiology.
FIG. 2 is a cross section of a stent strut that has been coated with a plastic material.
FIG. 3 is a cross section of a stent strut that has been coated with a plastic material into which an anti-restenosis drug has been placed.
FIG. 4 is a cross section of a stent strut that is coated with a plastic material and has had an anti-restenosis placed onto the outer surface of the plastic coating.
FIG. 5 is a cross section of a stent strut onto which an anti-restenosis drug coating has been placed onto the outer surface of the stent that is deployed against an arterial wall and an anti-thrombogenic coating has been placed on the stent's inner surface that is in contact with blood.
FIG. 6A is a longitudinal cross section of an artery into which a stent has been deployed.
FIG. 6B is a longitudinal cross section of an artery into which a stent has been deployed and a balloon catheter has been placed that provides local delivery of an anti-restenosis drug.

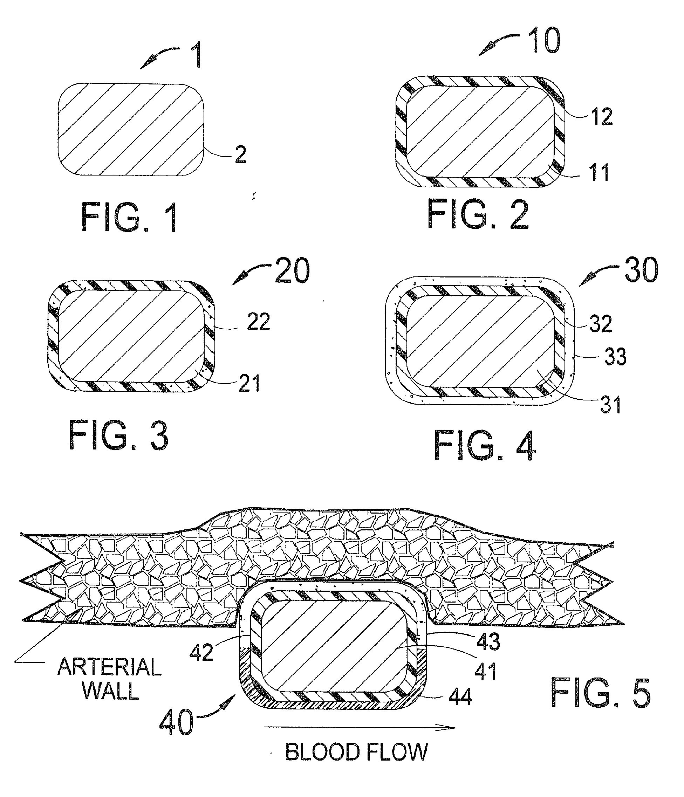

Referring to the drawings, FIG. 1 shows a cross section of a strut 2 of a typical prior art metal stent 1 that is used for placement into an artery of a human subject. These stents are typically laser cut from a thin-walled metal tube and then electro-chemically polished to round the edges of the struts.

FIG. 2 shows a typical stent strut 11 which is part of a stent 10. The strut 11 is coated with a flexible plastic 12 such as paralene or any one of a large variety elastomer materials such as silicone rubber, polyurethane, polyethylene, Nylon, PTFE, etc.

FIG. 3 is a cross section of a stent strut 21 that is part of a stent 20. The strut 21 is coated with a flexible plastic coating 22 into which one or more drugs can be diffused. One class of drugs would be an anti-restenosis drug that is selected from the group that includes, Alkeran, Cytoxan, Leukeran, Cis-platinum, BiCNU, Adriamycin, Doxorubicin, Cerubidine, Idamycin, Mithracin, Mutamycin, Fluorouracil, Methotrexate, Thoguanine, Toxotere, Etoposide, Vincristine, Irinotecan, Hycamptin, Matulane, Vumon, Hexalin, Hydroxyurea, Gemzar, Oncovin, Etophophos, tacrolimus (FK506), and the following analogs of sirolimus: SDZ-RAD, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy, 2-desmethyl and proline.

A second class of drugs that could be impregnated into the plastic coating 22 is an anti-thrombogenic drug such as heparin. It is of course possible to diffuse both an anti-restenosis drug and an anti-thrombogenic drug into the plastic coating 22.

FIG. 4 is a cross section of a stent strut 31 that is part of a stent 30. The strut 31 is coated with a flexible plastic 32 that is coated on its exterior surface with either or both an anti-restenosis drug and/or an anti-thrombogenic drug. As with the stent 20, the anti-restenosis drug would be selected from the group that includes, Alkeran, Cytoxan, Leukeran, Cis-platinum, BiCNU, Adriamycin, Doxorubicin, Cerubidine, Idamycin, Mithracin, Mutamycin, Fluorouracil, Methotrexate, Thoguanine, Toxotere, Etoposide, Vincristine, Irinotecan, Hycamptin, Matulane, Vumon, Hexalin, Hydroxyurea, Gemzar, Oncovin, Etophophos, tacrolimus (FK506), and the following analogs of sirolimus: SDZ-RAD, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy, 2-desmethyl and proline.

FIG. 5 is a cross section of a stent strut 41 of a stent 40, the stent 40 being deployed so that its outer surface is placed against the arterial wall of a human subject. The outer portion 43 of the stent 40 being an outer surface of the stent 40 that is in contact with the arterial wall and the inner portion 44 of the stent 40 being in contact with blood that flows through the arterial lumen. The strut 41 is coated with a flexible plastic material 42 onto which is coated an anti-restenosis drug on the outer portion 43. By releasing the anti-restenosis drug from the outer portion 43 into the arterial wall, the rate of restenosis for the stent 40 will be considerably reduced. It should also be understood that the anti-restenosis drug could be a single drug or a combination of drugs selected from the group that includes, Alkeran, Cytoxan, Leukeran, Cis-platinum, BiCNU, Adriamycin, Doxorubicin, Cerubidine, Idamycin, Mithracin, Mutamycin, Fluorouracil, Methotrexate, Thoguanine, Toxotere, Etoposide, Vincristine, Irinotecan, Hycamptin, Matulane, Vumon, Hexalin, Hydroxyurea, Gemzar, Oncovin, Etophophos, tacrolimus (FK506), and the following analogs of sirolimus: SDZ-RAD, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy, 2-desmethyl and proline.

The inner portion 44 on the inner surface of the stent 40 is coated with an anti-thrombogenic drug that is designed to decrease the rate of acute and subacute thrombosis that can result when the bare metal of the stent 40 is exposed to blood flow.

Although the strut 41 of FIG. 5 shows the anti-restenosis drug and the anti-thrombogenic drug coated onto the exterior surface of the plastic coating 42, it should be understood that one or both of the drugs could be either coated onto the surface of the plastic coating 42 or one or both of the drugs could be diffused into the plastic coating 42. It should also be understood that one of the drugs could be placed entirely around the stent strut 41 while the other drug occupies either the outer portion 43 or the inner portion 44.

To fabricate a stent such as the stent 40, the metal struts 41 could be formed in a conventional manner, for example by laser cutting of a thin-walled metal tube. The plastic coating 42 could be formed in a conventional manner, for example by vapour deposition (for parylene) or by dipping (silicone rubber). The outer portion 43 could be formed by expanding a balloon at low pressure within the stent 40 and then placing the anti-restenosis drug into the outer portion 43 either by coating the plastic coating 42 or diffusing the drug into the plastic coating 42. The balloon would then be deflated and removed and the stent 40 placed into an elastic tube that would make firm contact with the outer portion 43 of the stent 40. One or more anti-thrombogenic drugs would then be made to flow through the tube until the inner portion 44 of the flexible plastic coating 42 was coated with the anti-thrombogenic drug or the drug was caused to diffuse into the plastic coating 42.

It should also be understood that when the stent is placed into the patient, the patient could also take either or both an anti-restenosis drug or an anti-thrombogenic drug by mouth, by injection or by any other means that would place the drug systemically throughout the patient's body. It is further understood that one type of drug could be placed on the stent while a second and/or third type could be systemically administered. It is further understood that either or both the drugs Plavix and/or aspirin could be given after stent implantation with or without any other drug.

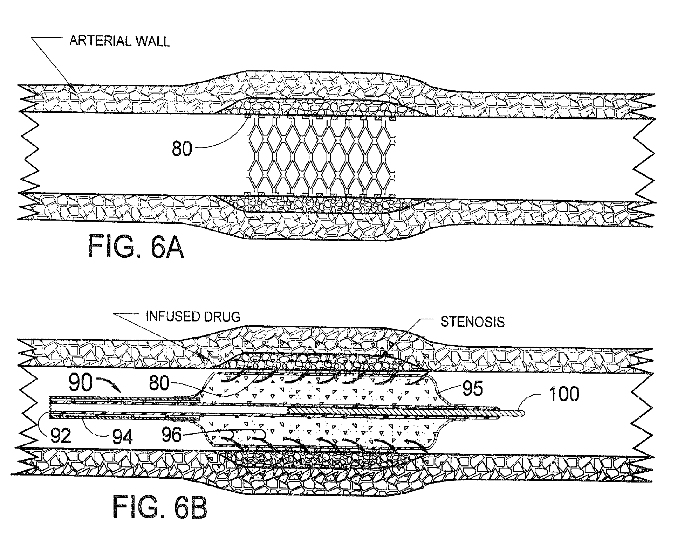

Another embodiment of the present invention is the use of an anti-restenosis drug that is delivered locally at the site where a stent has been deployed in a stenosis. FIG. 6A shows a stent 80 that has been deployed into an arterial stenosis. FIG. 6B shows a balloon catheter 90 having an inner shaft 92, an outer shaft 94 and a balloon 95. The balloon 95 has a multiplicity of tiny holes through which an anti-restenosis drug can be local delivered into the region that surrounds the stent 80. The arrows 96 show the direction and placement of drug injection into the tissue that surrounds the stent 80. The drug to be injected would be selected from the group that includes Alkeran, Cytoxan, Leukeran, Cis-platinum, BiCNU, Adriamycin, Doxorubicin, Cerubidine, Idamycin, Mithracin, Mutamycin, Fluorouracil, Methotrexate, Thoguanine, Toxotere, Etoposide, Vincristine, Irinotecan, Hycamptin, Matulane, Vumon, Hexalin, Hydroxyurea, Gemzar, Oncovin, Etophophos, tacrolimus (FK506), and the following analogs of sirolimus: SDZ-RAD, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy, 2-desmethyl and proline.

Although the stent 80 could be a conventional metal stent, ideally the stent 80 would be coated with an anti-restenosis drug so as to decrease the possibility of acute or subacute thrombosis.

## Claims

1. A stent for implantation into an artery of a human subject, the stent comprising:
a thin-walled, lace-like, metal structure formed into the general shape of a cylindrical tube; and
a drug coating on the surface of the stent, the drug being an anti-restenosis drug selected from the group that includes, Alkeran, Cytoxan, Leukeran, BiCNU, Cerubidine, Fluorouracil, Methotrexate, Toxotere, Irinotecan, Hycamptin, Matulane, Vumon, Hexalin, Gemzar, Oncovin, Etophophos.

2. The stent of claim 1 wherein the stent is coated with a plastic material that is selected from the group that includes parylene, silicone rubber, polyurethane, polyethylene, Nylon and PTFE.

3. The stent of claim 2 wherein the anti-restenosis drug is diffused into the plastic coating.

4. The stent of claim 2 wherein the anti-restenosis is coated onto the exterior surface of the plastic coating.

5. The stent of claim 1 wherein the stent is also coated with an anti-thrombogenic drug.

6. A stent for implantation into an artery of a human subject, the stent comprising:
a thin-walled, lace-like, metal structure formed into the general shape of a cylindrical tube;
an anti-restenosis drug coating on that outer surface of the stent that is placed in contact with the wall of the artery when the stent is deployed, the anti-restenosis drug being selected from the group that includes, Alkeran, Cytoxan, Leukeran, BiCNU, Cerubidine, Fluorouracil, Methotrexate, Toxotere, Irinotecan, Hycamptin, Matulane, Vumon, Hexalin, Gemzar, Oncovin, Etophophos; and
an anti-thrombogenic drug placed on the inner surface of the stent, the inner surface being exposed to blood flow within the lumen of the artery.

7. The stent of claim 6 wherein the stent is coated with a plastic material that is selected from the group that includes parylene, silicone rubber, polyurethane, polyethylene, Nylon and PTFE.

8. The stent of claim 7 wherein the anti-restenosis drug is diffused into the plastic coating.

9. The stent of claim 7 wherein the anti-restenosis is coated onto at least part of the exterior surface of the plastic coating.

10. The stent of claim 7 wherein the anti-thrombogenic drug is diffused into at least part of the plastic coating.

11. The stent of claim 7 wherein the anti-thrombogenic is coated onto at least part of the exterior surface of the plastic coating.

12. An assembly which comprises a stent and a balloon catheter adapted for local delivery of an anti-restenosis drug, in which:
the stent is in the form of a thin-walled, lace-like, generally cylindrical tube that is deployed radially outward against the wall of an artery in a human subject; and
the balloon catheter is adapted for local delivery of an anti-restenosis drug, the balloon catheter having a balloon located at a distal section of the balloon catheter, the balloon having a multiplicity of tiny holes through which the drug can be injected into the arterial wall in the vicinity of the stent;
the assembly including a anti-restenosis drug that is injected into the arterial wall being selected from the group that includes Alkeran, Cytoxan, Leukeran, BiCNU, Cerubidine, Fluorouracil, Methotrexate, Toxotere, Irinotecan, Hycamptin, Matulane, Vumon, Hexalin, Gemzar, Oncovin, Etophophos.

13. An assembly as claimed in claim 12, which includes an anti-thrombogenic drug coating on the surface of the stent.
